# EUROPEAN PATENT APPLICATION

(11) **EP 4 825 379 A1**
(43) Date of publication of application: **21.10.2026**
(21) Application number: 24902918.2
(22) Date of filing: 12.12.2024
(51) Int. Cl.: A61K 31/454, A61K 31/4545, A61K 31/46, A61P 37/02, A61P 25/28, A61P 25/16, A61P 29/00, A61P 25/00, A61P 25/08

(54) **USE OF 1-(3-AMINOPROPYL) SUBSTITUTED CYCLIC AMINE COMPOUND**

(30) Priority: 13.12.2023 CN 202311711850
(71) Applicant: Shanghai Institute of Materia Medica, Chinese Academy of Sciences, Shanghai 201203 (CN); Beijing Tiantan Hospital Capital Medical University, Beijing 100070 (CN)
(72) Inventor: LIU, Hong, Shanghai 201203 (CN); SHI, Fudong, Beijing 100070 (CN); WANG, Jiang, Shanghai 201203 (CN); SHI, Kaibin, Beijing 100070 (CN)
(74) Representative: Mewburn Ellis LLP
(86) International application number: PCT/CN2024/138891
(87) International publication number: WO 2025/124501

(57) **Abstract**

The present invention relates to pharmaceutical use of a 1-(3-aminopropyl) substituted cyclic amine compound. Specifically, the present invention relates to use of a 1-(3-aminopropyl) substituted cyclic amine compound represented by general formula (I) or a pharmaceutically acceptable salt thereof, a solvate thereof, an optically pure isomer thereof, a stereoisomer thereof, or a mixture thereof in the preparation of a drug for treating and/or preventing multiple sclerosis and related diseases thereof.

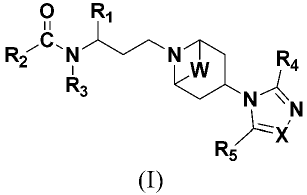

## Description

### TECHNICAL FIELD

The present invention relates to the pharmaceutical field, specifically to the use of 1-(3-aminopropyl) substituted cyclic amine compounds in the preparation of medicaments for treating and/or preventing multiple sclerosis and related diseases thereof.

### BACKGROUND

Multiple sclerosis (MS) is an immune-mediated disease characterized by chronic inflammatory demyelination of the central nervous system, mainly affecting the spinal cord and white matter of the brain, accompanied by inflammatory infiltration, demyelination, and glial cell damage, etc. According to the progression of the disease, multiple sclerosis can be classified into relapsing remitting MS (RRMS), primary progressive MS (PPMS), and secondary progressive MS (SPMS). The clinical symptoms of MS are often manifested as limb weakness, visual disturbances, and dizziness, etc., reaching their symptom peak within days and leading to paralysis or blindness in severe cases. When multiple sclerosis enters the late stage, cognitive dysfunction and mental abnormalities may also occur. Currently, there are approximately 2.3 million cases of MS worldwide, with an incidence rate of about 50 per million people in China, and a significant upward trend in recent years. The average survival time for patients with multiple sclerosis is 40 years. They suffer greatly from the disease and its treatment, and face a series of problems such as progressive disability and psychosocial adaptation for a long time. This not only brings great suffering to patients, but also imposes an extremely heavy economic burden on society.

At present, the pathogenesis of multiple sclerosis remains unclear, but factors involved include the central nervous system, immune system, nutrition, and environment. Demyelination, a major pathological marker of multiple sclerosis, is likely caused by T cell and B cell-mediated immune responses. During the process of multiple sclerosis, the peripheral immune system mediated by T cells and B cells is activated, releasing a large amount of inflammatory factors, attacking the blood xiufbrain barrier (BBB), and infiltrating the central nervous system. The changes in the inflammatory microenvironment of the central nervous system lead to the proliferation of glial cells and excessive secretion of inflammatory factors, thereby damaging oligodendrocytes, causing necrosis and apoptosis, and triggering the shedding of myelin sheaths. At present, drugs used to treat multiple sclerosis only have a repair therapy effect. There are over 10 types of multiple sclerosis corrective treatment drugs on the market abroad, mainly by inhibiting peripheral immune responses to reduce disease recurrence and delay disability. Among them, fingolimod, as a sphingosine-1-phosphate receptor agonist, binds to S1P receptors on lymphocytes to prevent their release from lymphoid tissue, thereby reducing their infiltration into the central nervous system to trigger multiple sclerosis. However, due to the diverse distribution of S1P in the human body, adverse reactions such as lymphocyte depletion, bradycardia, atrioventricular block, and macular edema are prone to occur after using Fingolimod. Currently, only interferon β-1a, β-1b, and teriflunomide are approved in China. Among them, teriflunomide is the main active metabolite of the antirheumatic drug leflunomide, it protects nervous system function by inhibiting dihydroorotate dehydrogenase, a key mitochondrial enzyme in the de novo pyrimidine synthesis pathway, thereby reducing the proliferation of activated T cells and B cells, lymphocyte infiltration in the central nervous system, and axonal loss. Due to the limited availability of drugs in the Chinese market for preventing the recurrence of multiple sclerosis and the lack of effective treatment methods to prevent axonal injury or myelin repair, patients with multiple sclerosis face significant treatment challenges.

It has been reported that CCR5 antagonists can be used to inhibit multiple sclerosis. However, current CCR5 antagonists for treating multiple sclerosis have problems such as long dosing cycles and slow onset of action, making it difficult for CCR5 antagonists to be practically applied in the preparation of drugs for the treatment of multiple sclerosis.

Therefore, there is an urgent need in this field to develop novel drugs that can effectively treat and/or prevent multiple sclerosis and related diseases thereof.

### SUMMARY OF THE INVENTION

The purpose of the present invention is to provide the use of 1-(3-aminopropyl) substituted cyclic amine compounds in the preparation of a medicament for treating and/or preventing multiple sclerosis and a related disease thereof.

In the first aspect of the present invention, it provides a use of a compound of formula (I), or a pharmaceutically acceptable salt, a solvate, an optically pure isomer, a stereoisomer, or a mixture thereof, in the preparation of a medicament for treating and/or preventing multiple sclerosis and a related disease thereof;
the compound of formula (I) has the following structure: 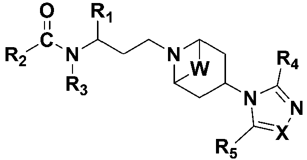 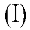
wherein,
W is absent or is -CH₂CH₂-; X is N or CR₆;
R₁ is selected from a 5~7 membered heteroaryl which is unsubstituted or substituted with 1-3 substituents, wherein the heteroaryl contains 1~3 heteroatoms selected from oxygen, sulfur, and nitrogen, and the substituent is each independently selected from halogen, C1~C4 linear or branched alkyl, C1~C4 linear or branched haloalkyl, C1~C4 linear or branched alkoxy, C1~C4 linear or branched haloalkoxy, -NR₁₀R₁₁, -C(=O)R₁₂, C1~C4 linear or branched alkanoyloxy, cyano, nitro, and hydroxyl, or two adjacent substituents, together with the carbon atoms to which they are attached, form a 5-7 membered ring;
R₁₀ and R₁₁ are each independently selected from H, C1~C4 linear or branched alkyl and - C(=O)R₁₃;
R₁₂ is selected from C1~C4 linear or branched alkyl, C1~C4 linear or branched alkoxy, hydroxy, amino (NH₂) and C1~C4 linear or branched alkylamino;
R₁₃ is selected from H and C1~C4 linear or branched alkyl;
R₂ is selected from the following groups which are unsubstituted or substituted with 1-3 substituents: C1~C6 linear or branched alkyl, C3~C7 cycloalkyl, 4~7 membered heterocyclyl, C6~C12 aryl, or 5~7 membered heteroaryl; wherein the substituent is selected from halogen, hydroxyl, C1~C4 linear or branched alkyl, C1~C4 linear or branched haloalkyl, C1~C4 linear or branched alkoxy, C1~C4 linear or branched alkylcarbonyl, C1~C4 linear or branched haloalkoxy, C1~C4 linear or branched alkylsulfonyl, C1~C4 linear or branched alkylsulfonyl carbamoyl, tetrazolyl, cyano, nitro, amino, carboxyl, phenyl and phenoxy;
R₃, R₄ and R₅ are each independently selected from hydrogen, C1~C6 linear or branched alkyl, C1~C6 linear or branched haloalkyl and C3~C7 cycloalkyl;
R₆ is selected from hydrogen and C1~C6 linear or branched alkyl;
or, R₅ and R₆ can be connected together with 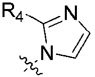 to form 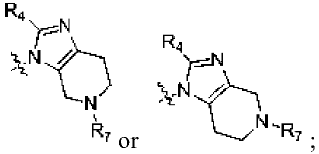
R₇ is selected from hydrogen, C(=O)R₈, C(=O)OR₈, C(=O)NR₈R₉, SO₂R₈ and the following groups substituted with 1-3 substituents: C1~C6 linear or branched alkyl, C3~C7 cycloalkyl, 4~7 membered heterocyclyl, benzyl, C6~C12 aryl and 5~7 membered heteroaryl; wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino, carboxyl;
R₈ and R₉ are each independently selected from hydrogen, and the following groups which are unsubstituted or substituted with 1-3 substituents: C1~C6 linear or branched alkyl, C3~C7 cycloalkyl, 4~7 membered heterocyclyl, benzyl, C6~C12 aryl and 5~7 membered heteroaryl; wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino, carboxyl.

In another preferred embodiment, the compound is selected from the group consisting of:

| Number | Structure | Number | Structure |
|---|---|---|---|
| DC521020 | 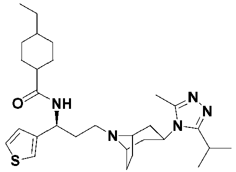 | DC521022 | 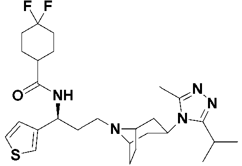 |
| DC521026 | 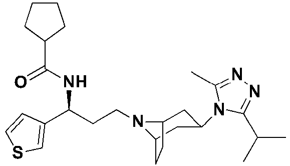 | DC521042 | 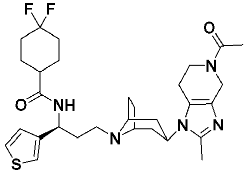 |
| DC521043 | 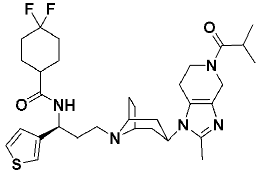 | DC521068 | 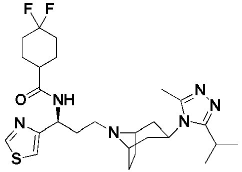 |
| DC521124 | 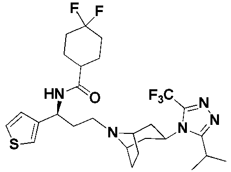 | DC521127 | 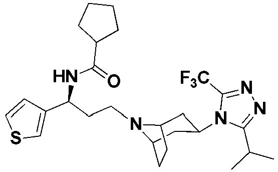 |
| DC521131 | 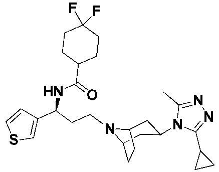 | DC521133 | 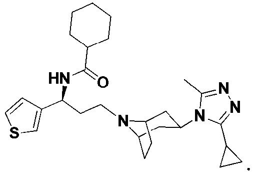 |

In another preferred embodiment, the multiple sclerosis is selected from the group consisting of: relapsing-remitting multiple sclerosis, secondary progressive multiple sclerosis, primary progressive multiple sclerosis, progressive-relapsing multiple sclerosis, or a combination thereof.

In another preferred embodiment, the multiple sclerosis related disease is selected from the group consisting of: neuromyelitis optica, acute disseminated encephalomyelitis, stroke, craniocerebral injury, epilepsy, Alzheimer's disease, Parkinson's disease, immune inflammatory response, and demyelinating injury.

In another preferred embodiment, the multiple sclerosis and a related disease thereof comprises: relapsing-remitting multiple sclerosis, secondary progressive multiple sclerosis, primary progressive multiple sclerosis, stroke, craniocerebral injury, epilepsy, immune inflammatory response, and demyelinating injury.

In another preferred embodiment, the compound is used for:
(1) repairing demyelination in the spinal cord of multiple sclerosis;
(2) reducing infiltrating immune cells in the spinal cord of multiple sclerosis;
(3) reducing myeloid skewing in the spinal cord of multiple sclerosis.

In another preferred embodiment, the pharmaceutically acceptable salt comprises conventional pharmaceutically acceptable salts formed by the reaction of the compound of the present invention with an inorganic or organic acid. For example, the conventional pharmaceutically acceptable salts can be prepared by reacting the compound of the present invention with an inorganic or organic acid, wherein the inorganic acid comprises hydrochloric acid, hydrobromic acid, sulfuric acid, nitric acid, aminosulfonic acid, and phosphoric acid, etc., and the organic acid comprises citric acid, tartaric acid, lactic acid, pyruvic acid, acetic acid, benzenesulfonic acid, p-toluenesulfonic acid, methanesulfonic acid, naphthalenesulfonic acid, ethanesulfonic acid, naphthalenedisulfonic acid, maleic acid, malic acid, malonic acid, fumaric acid, succinic acid, propionic acid, oxalic acid, trifluoroacetic acid, stearic acid, pamoic acid, hydroxymaleic acid, phenylacetic acid, benzoic acid, salicylic acid, glutamic acid, ascorbic acid, p-aminobenzenesulfonic acid, 2-acetoxybenzoic acid, and hydroxy-14-ethanesulfonic acid, etc.; or sodium salt, potassium salt, calcium salt, aluminum salt, or ammonium salt formed by the compound of the present invention with inorganic bases; or methylamine salt, ethylamine salt, or ethanolamine salt formed by the compound of the present invention with organic bases.

In another preferred embodiment, the administration method of the compound is selected from the group consisting of oral, rectal, and parenteral administration.

In another preferred embodiment, the method of the parenteral administration is selected from the group consisting of intravenous, intramuscular, or subcutaneous administration.

In another preferred embodiment, the formulation for the oral administration is selected from the group consisting of capsules, tablets, pills, powders, granules, emulsions, solutions, suspensions, syrups, and tinctures.

The second aspect of the present invention provides a use of a pharmaceutical composition in the preparation of a medicament for treating and/or preventing multiple sclerosis and a related disease thereof;
and the pharmaceutical composition comprises:
   (i) a first active ingredient, wherein the first active ingredient is selected from the group consisting of: the compound of formula (I), or a pharmaceutically acceptable salt, a solvate, an optically pure isomer, and a stereoisomer thereof;
   (ii) optionally, a second active ingredient, wherein the second active ingredient is selected from the group consisting of: interferon β-1a, interferon β-1b, teriflunomide, fingolimod, copaxone, ofatumumab, or a combination thereof; and
   (iii) a pharmaceutically acceptable carrier;
the compound of formula (I) is as defined in the first aspect of the present invention.

In another preferred embodiment, the compound of formula (I) is selected from the group consisting of:

| Number | Structure | Number | Structure |
|---|---|---|---|
| DC521020 | 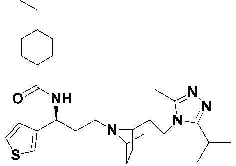 | DC521022 | 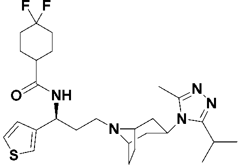 |
| DC521026 | 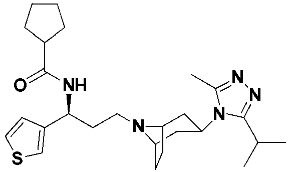 | DC521042 | 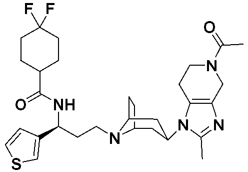 |
| DC521043 | 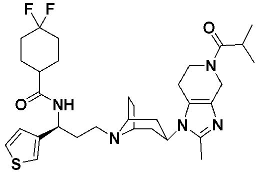 | DC521068 | 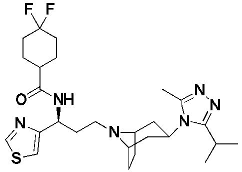 |
| DC521124 | 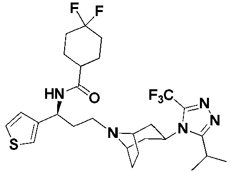 | DC521127 | 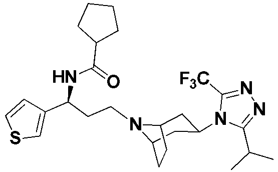 |
| DC521131 | 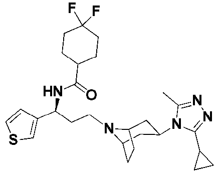 | DC521133 | 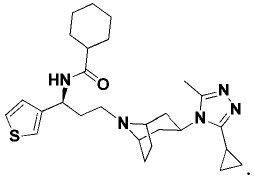 |

In another preferred embodiment, the content of the first active ingredient in the pharmaceutical composition is 0.01-99 wt%, more preferably 0.1-90 wt%, based on the total weight of the pharmaceutical composition.

In another preferred embodiment, the compound of formula (I) is used as a single component in combination with other effective treatments (surgical treatment, radiotherapy, etc.) of multiple sclerosis for use in the comprehensive treatment of multiple sclerosis.

The third aspect of the present invention provides a method for treating and/or preventing multiple sclerosis and a related disease thereof, comprising administering a medically effective amount of the compound of formula (I) or a pharmaceutically acceptable salt, a solvate, an optically pure isomer, a stereoisomer, or a mixture thereof to a subject in need;
wherein the compound of formula (I) is as defined in the first aspect of the present invention.

In another preferred embodiment, the subject is a primate mammal, such as human.

The present invention relates to the preparation of a medicament for treating and/or preventing multiple sclerosis using 1-(3-aminopropyl) substituted cyclic amine compound of general formula (I) or a pharmaceutically acceptable salt, a solvate, a stereoisomer, a tautomer, a prodrug, and a mixture thereof as an active ingredient.

The medicament can be introduced into the body, such as muscle, intradermal, subcutaneous, vein, or mucous tissue, via injection, spray, nasal drops, eye drops, osmosis, absorption, or physical or chemical mediated methods; or it can be introduced into the body via mixed with or encapsulated by other substances. When necessary, one or more pharmaceutically acceptable carriers may be added to the above-mentioned medicament.

The carrier comprises common pharmaceutical diluent, excipient, filler, binder, wetting agent, disintegrant, absorption enhancer, surfactant, adsorbent, lubricant, etc.

1-(3-aminopropyl) substituted cyclic amine compounds of general formula (I) or a pharmaceutically acceptable salt, an ester, a solvate, a stereoisomer, a tautomer, or a prodrug as an active ingredient can be used alone or in combination or formulated with other drugs, excipients, etc. into various dosage forms, including but not limited to tablets, powders, pills, injections, capsules, films, suppositories, ointments, and granules, etc. All of the above dosage forms can be prepared using conventional methods in the pharmaceutical field.

It should be understood that, within the scope of the present invention, each technical feature of the present invention described above and the technical features specifically described below (such as in the examples) can be combined with each other to form new technical solutions or in another preferred embodiment. The various features disclosed in the specification can be replaced by any alternative features that provide the same, equivalent, or similar purpose. Due to space limitations, they will not be described in detail here.

### DESCRIPTION OF THE DRAWINGS

Figure 1 shows the neurological deficit scores and weight changes of mice in the model administration group (EAE+DC521022) and the model control group (EAE+Vehicle) after immunization; Figure 1A shows intraperitoneal injection of 50 mg/kg DC521022 and saline on the day of modeling; Figure 1B shows intraperitoneal injection of 50 mg/kg DC521022 and saline after the onset of symptoms in mice; Figure 1C shows gavage administration of 50 mg/kg DC521022 and saline on the day of modeling; Figure 1D shows gavage administration of 50 mg/kg DC521022 and saline after the onset of symptoms in mice.
Figure 2 shows H&E and Luxol fast blue staining of spinal cord tissue sections from mice in the DC521022 treatment group and the saline treatment group 14 days and 28 days after administration, respectively.
Figure 3 shows the percentage of demyelination and inflammatory cell infiltration in the spinal cord of mice in the DC521022 treatment group and the saline treatment group.
Figure 4 shows the results of flow cytometry analysis of the myeloid skewing in mouse bone marrow on Day 14 and Day 28 after DC521022 administration. Figure 4A shows the myeloid shift in mouse bone marrow analyzed by flow cytometry; Figure 4B is a schematic diagram of the changes in the proportion and quantity of femoral hematopoietic stem cells (HSCs) after 14 days; Figure 4C is a schematic diagram of the changes in the proportion and quantity of hematopoietic stem cells after 14 days.
Figure 5 shows the clinical scoring results of the EAE model group treated with the CCR5 antagonist DAPTA.

### DETAILED DESCRIPTION OF THE INVENTION

Through extensive and intensive research and numerous screenings, for the first time, the inventors have unexpectedly developed a class of active ingredients that can effectively inhibit multiple sclerosis and a related disease thereof, namely the compound represented by general formula (I) or a pharmaceutically acceptable salt, an enantiomer, a diastereomer, or a racemate thereof. Experimental results demonstrate that the active ingredient of the present invention can efficiently repair demyelination in the spinal cord affected by multiple sclerosis, reduce infiltrating immune cells and myeloid skewing in the spinal cord, thereby treating and/or preventing multiple sclerosis. The present invention was completed on this basis.

### Terms

The "alkyl" refers to a linear or branched saturated aliphatic hydrocarbon group. For example, "C1-8 alkyl" refers to linear alkyl and branched alkyl that comprise 1 to 8 carbon atoms, including but not limited to methyl, ethyl, n-propyl, isopropyl, n-butyl, isobutyl, tert-butyl, sec-butyl, n-pentyl, etc.

The "cycloalkyl" refers to a saturated or partially unsaturated monocyclic or polycyclic cyclic hydrocarbon substituent. For example, "C3-8 cycloalkyl" refers to a cycloalkyl containing 3 to 8 carbon atoms, and is divided into monocyclic cycloalkyl and polycyclic cycloalkyl. Monocyclic cycloalkyl includes, but not limited to, cyclopropyl, cyclobutyl, cyclopentyl, cyclopentenyl, cyclohexyl, cyclohexenyl, cyclohexadienyl, cycloheptyl, cycloheptatrienyl, and cyclooctyl, etc. Polycyclic cycloalkyl includes spiro, fused, and bridged cycloalkyls.

The "heterocyclyl" refers to a saturated or partially unsaturated monocyclic or polycyclic cyclic hydrocarbon substituent, wherein one or more ring atoms are selected from nitrogen, oxygen, or S.

The "aryl" refers to an all carbon monocyclic or fused polycyclic (i.e., a ring that shares adjacent carbon atom pairs) group, a polycyclic (i.e., a ring with adjacent carbon atom pairs) group having a conjugated π-electron system, including but not limited to phenyl and naphthyl.

The "heteroaryl" refers to a heteroaromatic system containing 1 to 4 heteroatoms, including nitrogen, oxygen and S. For example, 5-7 membered heteroaryl refers to a heteroaromatic system containing 5-7 ring atoms, and 5-10 membered heteroaryl refers to a heteroaromatic system containing 5-10 ring atoms, including but not limited to furyl, thienyl, pyridyl, pyrrolyl, N-alkylpyrrolyl, pyrimidinyl, pyrazinyl, imidazolyl, tetrazolyl, etc.

The "alkoxy" refers to -O-(alkyl), wherein alkyl is as defined above. For example, "C1-8 alkoxy" refers to a alkyloxy group containing 1-8 carbons, including but not limited to methoxy, ethoxy, propoxy, butoxy, etc. The present invention will be further illustrated below with reference to specific embodiments.

The "pharmaceutically acceptable carrier" refers to one or more compatible solid or liquid fillers or gel substances that are suitable for human use and must have sufficient purity and sufficiently low toxicity. "Compatible" here means that the components of the composition can be mixed with the compound of general formula (I) of the present invention, a pharmaceutically acceptable salt or a solvate thereof, as well as with each other, without significantly reducing the efficacy of the active ingredient. Some examples of pharmaceutically acceptable carriers include cellulose and derivatives thereof (such as sodium carboxymethyl cellulose, sodium ethyl cellulose, cellulose acetate, etc.), gelatin, talc, solid lubricants (such as stearic acid, magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agents (such as sodium dodecyl sulfate), coloring agents, flavoring agents, stabilizers, antioxidants, preservatives, pyrogen-free water, etc.

Solid dosage forms for oral administration include capsules, tablets, pills, powders, and granules.

Liquid dosage forms for oral administration include pharmaceutically acceptable emulsions, solutions, suspensions, syrups, or tinctures. In addition to the active ingredient, the liquid dosage forms may contain inert diluents conventionally used in the art, such as water or other solvents, solubilizers and emulsifiers, e.g., ethanol, isopropanol, ethyl carbonate, ethyl acetate, propylene glycol, 1,3-butanediol, dimethylformamide, and oils, particularly cottonseed oil, peanut oil, corn germ oil, olive oil, castor oil, and sesame oil, or mixtures thereof. Besides these inert diluents, the composition may also contain adjuvants such as wetting agents, emulsifiers and suspending agents, sweeteners, flavoring agents, and perfumes.

In addition to the active ingredient, the suspension may contain suspending agents, such as ethoxylated isooctadecanol, polyoxyethylene sorbitol and dehydrated sorbitol esters, microcrystalline cellulose, aluminum methoxide and agar, or mixtures of these substances, etc.

There are no particular limitations on the administration of the compound or pharmaceutical composition of formula (I) of the present invention. Representative administration methods include (but not limited to): oral, rectal, parenteral (intravenous, intramuscular or subcutaneous) administration, etc.

Compositions for parenteral injection may comprise physiologically acceptable sterile aqueous or anhydrous solutions, dispersions, suspensions, or emulsions, and sterile powders for reconstitution into sterile injectable solutions or dispersions. Suitable aqueous and non-aqueous carriers, diluents, solvents, or excipients include water, ethanol, polyols, and suitable mixtures thereof.

### Active ingredient

The compound of formula (I) of the present invention has the following structure: 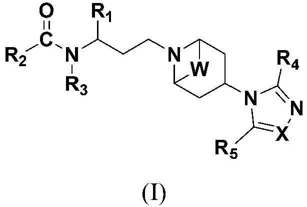 wherein, each group is as defined above.

In another preferred embodiment, the compound is in S configuration or R configuration, preferably in S configuration.

In another preferred embodiment, R₁ is selected from the following groups which are unsubstituted or substituted with 1-3 substituents: 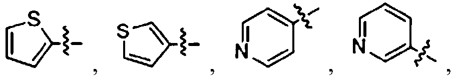 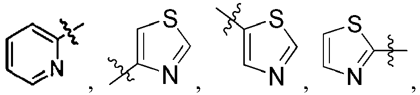 wherein the substituent is selected from halogen, C1~C4 linear or branched alkyl, C1~C4 linear or branched haloalkyl, C1~C4 linear or branched alkoxy, - NR₁₀R₁₁, -C(=O)R₁₂, C1~C4 linear or branched alkyl carbonyloxy, C1~C4 linear or branched haloalkoxy, cyano, nitro, and hydroxyl, or two adjacent substituents, together with the carbon atoms to which they are attached, form a 5~7 membered ring; preferably, the substituent is selected from halogen, C1~C2 alkyl, C1~C2 haloalkyl, C1~C2 alkoxy, NR₁₀R₁₁, -C(=O)R₁₂, C1~C2 alkyl carbonyloxy, C1~C2 haloalkoxy, cyano, nitro, and hydroxyl, or two adjacent substituents, together with the carbon atoms to which they are attached, form a 5-7 membered carbocyclyl, 5-7 membered heteroaryl, or 5-7 membered heterocyclyl; preferably, the substituent is selected from halogen, methyl, methoxy, ethyl, amino, hydroxyl, cyano, nitro, acetyl, formamido, acetamido, carbamoyl, N-methylcarbamoyl, N,N-dimethylcarbamoyl, formyloxy, acetoxy, methoxycarbonyl, trifluoromethyl, and trifluoromethoxy, or two adjacent substituents, together with the carbon atoms to which they are attached, form a phenyl, a cyclopentenyl, or a dioxolenyl.

In another preferred embodiment, R₁₀ and R₁₁ are each independently selected from H, C1~C2 alkyl and -C(=O)R₁₃.

In another preferred embodiment, R₁₂ is selected from C1~C2 alkyl, C1~C2 alkoxy, hydroxyl, amino (NH₂), and C1~C2 alkylamino.

In another preferred embodiment, R₁₃ is selected from H and C1~C2 linear or branched alkyl.

In another preferred embodiment, R₂ is selected from the following groups which are unsubstituted or substituted with 1-3 substituents: C1~C4 linear or branched alkyl, C3~C7 cycloalkyl, 4~7 membered heterocyclyl, and phenyl, wherein the substituent is selected from halogen, hydroxyl, C1~C4 linear or branched alkyl, C1~C4 linear or branched haloalkyl, C1~C4 linear or branched alkoxy, C1~C4 linear or branched alkyl carbonyl, C1~C4 linear or branched haloalkoxy, C1~C4 linear or branched alkylsulfonyl, C1~C4 linear or branched alkylsulfonyl carbamoyl, tetrazolyl, cyano, nitro, amino, carboxyl, phenyl, halophenyl, phenoxy, and halophenoxy.

In another preferred embodiment, R₂ is selected from C1~C4 linear or branched alkyl, cyclopropyl, cyclobutyl, cyclopentyl, cyclohexyl, tetrahydropyran-4-yl, 1-methylpiperidin-4-yl, 1-acetylpiperidin-4-yl, 1-methanesulfonylpiperidin-4-yl, 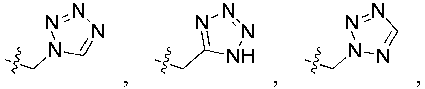 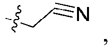 4-fluorobenzyl, phenyl, difluorocyclohexyl (such as 4,4-difluoroyclohexyl), ethyl cyclohexyl and phenoxymethyl.

In another preferred embodiment, R₃, R₄ and R₅ are each independently selected from hydrogen, C1~C4 linear or branched alkyl and C3~C7 cycloalkyl.

In another preferred embodiment, R₃, R₄ and R₅ are each independently selected from hydrogen, methyl, trifluoromethyl, ethyl, n-propyl, isopropyl, n-butyl, sec-butyl, tert-butyl, cyclopropyl, cyclobutyl, cyclopentyl and cyclohexyl.

In another preferred embodiment, R₃, R₄ and R₅ are each independently selected from hydrogen, methyl, ethyl, n-propyl, isopropyl, n-butyl, sec-butyl, tert-butyl and cyclopropyl.

In another preferred embodiment, R₆ is selected from hydrogen and C1~C4 linear or branched alkyl, more preferably selected from hydrogen, methyl and ethyl.

Alternatively, R₅ and R₆ can be connected together with 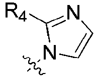 to form 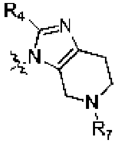 or

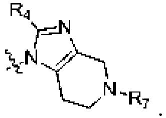

In another preferred embodiment, R₇ is selected from hydrogen, C(=O)R₈, C(=O)OR₈, C(=O)NR₈R₉, SO₂R₈, and the following groups substituted with 1-3 substituents: C1~C4 linear or branched alkyl, C3~C7 cycloalkyl, 4~7 membered heterocyclyl, benzyl, and phenyl, wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino, and carboxyl.

In another preferred embodiment, R₇ is selected from C(=O)R₈, C(=O)OR₈ and SO₂R₈;
R₈ and R₉ are each independently selected from hydrogen and the following groups which are unsubstituted or substituted with 1-3 substituents: C1~C4 linear or branched alkyl, C1~C4 linear or branched haloalkyl, C3~C7 cycloalkyl, 4~7 membered heterocyclyl, benzyl, phenyl and 5~7 membered heteroaryl, wherein the substituents are selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino and carboxyl, preferably selected from halogen, hydroxyl, methoxy, ethoxy, methyl, ethyl, trifluoromethyl, trifluoromethoxy, cyano, nitro, amino and carboxyl.

In another preferred embodiment, R₈ and R₉ are each independently selected from hydrogen, C1~C4 linear or branched alkyl, C1~C4 linear or branched haloalkyl, C3~C7 cycloalkyl, benzyl and phenyl.

In another preferred embodiment, R₈ and R₉ are each independently selected from methyl, ethyl, n-propyl, cyclopropyl, isopropyl, n-butyl, sec-butyl, and tert-butyl.

In another preferred embodiment, it provides 1-(3-aminopropyl) substituted cyclic amine compounds represented by general formula (II), a pharmaceutically acceptable salt, an enantiomer, a diastereomer, a racemate, or a mixture thereof: 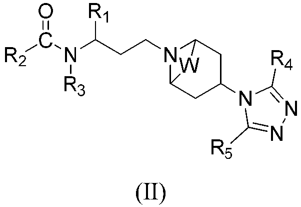 wherein, R₁, R₂, R₃, R₄, R₅, and W are as described in general formula (I).

In another preferred embodiment, in general formula (II), R₁ is selected from the following groups which are unsubstituted or substituted with 1-3 substituents: 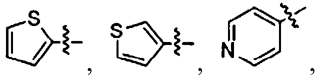 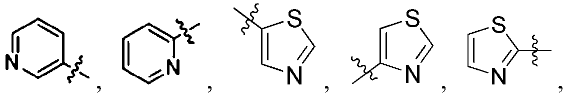 wherein the substituent is selected from halogen, C1~C4 linear or branched alkyl, C1~C4 linear or branched haloalkyl, C1~C4 linear or branched alkoxy, C1~C4 linear or branched alkyl carbonyloxy, C1~C4 linear or branched haloalkoxy, NR₁₀R₁₁, -C(=O)R₁₂, cyano, nitro, and hydroxyl, or two adjacent substituents, together with the carbon atoms to which they are attached, form a 5-7 membered ring; preferably, the substituent is selected from halogen, C1~C2 alkyl, C1~C2 haloalkyl, C1~C2 alkyl carbonyloxy, C1~C2 alkoxy, C1~C2 haloalkoxy, NR₁₀R₁₁, -C(=O)R₁₂, cyano, nitro, and hydroxyl, or two adjacent substituents, together with the carbon atoms to which they are attached, form a 5-7 membered carbocyclyl, 5-7 membered heteroaryl, or 5-7 membered heterocyclyl; preferably, the substituent is selected from halogen, methyl, trifluoromethyl, trifluoromethoxy, methoxy, ethyl, amino, cyano, nitro, acetyl, formamido, acetamido, carbamoyl, N-methylcarbamoyl, N,N-dimethylcarbamoyl, acetyloxy, formyloxy, and methoxycarbonyl, or two adjacent substituents, together with the carbon atoms to which they are attached, form a phenyl, a cyclopentenyl, or a dioxolenyl;
R₁₀ and R₁₁ are each independently selected from H, C1~C4 linear or branched alkyl and - C(=O)R₁₃; preferably, R₁₀ and R₁₁ are each independently selected from H, C1~C2 alkyl and - C(=O)R₁₃;
R₁₂ is selected from C1~C4 linear or branched alkyl, C1~C4 linear or branched alkoxy, hydroxy, amino (NH₂) and C1~C4 linear or branched alkylamino; preferably, R₁₂ is selected from C1~C2 alkyl, C1~C2 alkoxy, hydroxy, amino (NH₂) and C1~C2 alkylamino.
R₁₃ is selected from H and C1~C4 linear or branched alkyl; preferably, R₁₃ is selected from H and C1~C2 linear or branched alkyl;
R₂ is selected from phenyl which is unsubstituted or substituted with 1-3 substituents, C1~C4 linear or branched alkyl, and C3~C7 cycloalkyl, wherein the substituent is selected from halogen, hydroxyl, C1~C4 linear or branched alkyl, C1~C4 linear or branched haloalkyl, C1~C4 linear or branched alkoxy, C1~C4 linear or branched alkyl carbonyl, C1~C4 linear or branched haloalkoxy, C1~C4 linear or branched alkylsulfonyl, C1~C4 linear or branched alkylsulfonyl carbamoyl, tetrazolyl, amino, phenyl, halophenyl, phenoxy, and halophenoxy.

In another preferred embodiment, R₂ is selected from methyl, ethyl, cyclopropyl, cyclobutyl, cyclopentyl, cyclohexyl, tetrahydropyran-4-yl, 1-methylpiperidin-4-yl, 1-acetylpiperidin-4-yl, 1-methanesulfonylpiperidin-4-yl, 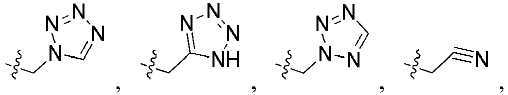 4-fluorobenzyl, phenyl, ethyl cyclohexyl and difluorocyclohexyl;
R₃, R₄ and R₅ are each independently selected from hydrogen and C1~C4 linear or branched alkyl.

In another preferred embodiment, R₃, R₄ and R₅ are each independently selected from hydrogen, methyl, ethyl, n-propyl, isopropyl, n-butyl, sec-butyl and tert-butyl.

In another preferred embodiment, R₃, R₄ and R₅ are each independently selected from hydrogen, methyl, ethyl, n-propyl and isopropyl.

In another preferred embodiment, it provides 1-(3-aminopropyl) substituted cyclic amine compound represented by general formula (III), a pharmaceutically acceptable salt, an enantiomer, a diastereomer, a racemate, or a mixture thereof: 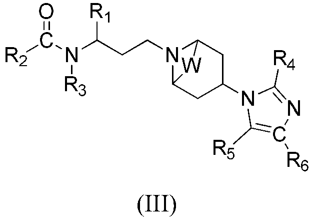 wherein, R₁, R₂, R₃, R₄, R₅, R₆, and W are as defined in general formula (I).

In general formula (III), preferably, R₁ is selected from the following groups which are unsubstituted or substituted with 1-3 substituents: 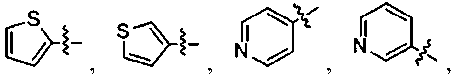 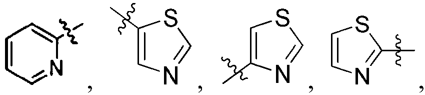 wherein the substituent is selected from halogen, C1~C4 linear or branched alkyl, C1~C4 linear or branched haloalkyl, C1~C4 linear or branched alkyl carbonyloxy, C1~C4 linear or branched alkoxy, C1~C4 linear or branched haloalkoxy, NR₁₀R₁₁, - C(=O)R₁₂, cyano, nitro, and hydroxyl, or two adjacent substituents, together with the carbon atoms to which they are attached, form a 5-7 membered ring; preferably, the substituent is selected from halogen, C1~C2 alkyl, C1~C2 haloalkyl, C1~C2 alkoxy, C1~C2 alkyl carbonyloxy, C1~C2 haloalkoxy, NR₁₀R₁₁, -C(=O)R₁₂, cyano, nitro, and hydroxyl, or two adjacent substituents, together with the carbon atoms to which they are attached, form a 5-7 membered carbocyclyl, 5-7 membered heteroaryl, or 5-7 membered heterocyclyl; preferably, the substituent is selected from halogen, methyl, trifluoromethyl, trifluoromethoxy, methoxy, ethyl, amino, cyano, nitro, acetyl, formamido, acetamido, carbamoyl, N-methylcarbamoyl, N,N-dimethylcarbamoyl, formyloxy, acetyloxy, and methoxycarbonyl, or two adjacent substituents, together with the carbon atoms to which they are attached, form a phenyl, a cyclopentenyl, or a dioxolenyl;
R₁₀ and R₁₁ are each independently selected from H, C1~C4 linear or branched alkyl and - C(=O)R₁₃; preferably, R₁₀ and R₁₁ are each independently selected from H, C1~C2 alkyl and - C(=O)R₁₃;
R₁₂ is selected from C1~C4 linear or branched alkyl, C1~C4 linear or branched alkoxy, hydroxy, amino (NH₂) and C1~C4 linear or branched alkylamino; preferably, R₁₂ is selected from C1~C2 alkyl, C1~C2 alkoxy, hydroxy, amino (NH₂) and C1~C2 alkylamino;
R₁₃ is selected from H and C1~C4 linear or branched alkyl; preferably, R₁₃ is selected from H and C1~C2 linear or branched alkyl;
R₂ is selected from C1~C4 linear or branched alkyl and C3~C7 cycloalkyl which are unsubstituted or substituted with 1-3 substituents, wherein the substituent is selected from halogen, hydroxyl, C1~C4 linear or branched alkyl, C1~C4 linear or branched haloalkyl, C1~C4 linear or branched alkoxy, C1~C4 linear or branched alkyl carbonyl, C1~C4 linear or branched haloalkoxy, C1~C4 linear or branched alkylsulfonyl, C1~C4 linear or branched alkylsulfonyl carbamoyl, tetrazolyl, cyano, and amino; preferably, R₂ is selected from methyl, ethyl, cyclopropyl, cyclobutyl, cyclopentyl, cyclohexyl, tetrahydropyran-4-yl, 1-methylpiperidin-4-yl, 1-acetylpiperidin-4-yl, 1-methanesulfonylpiperidin-4-yl, 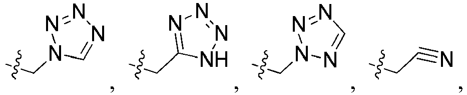 difluorocyclohexyl;
R₃ and R₄ are each independently selected from hydrogen and C1~C4 linear or branched alkyl; preferably, R₃ and R₄ are each independently selected from hydrogen, methyl, ethyl, n-propyl, isopropyl, n-butyl, sec-butyl and tert-butyl; preferably, R₃ and R₄ are each independently selected from hydrogen, methyl and ethyl;
R₅ and R₆ can be connected together with 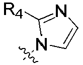 to form 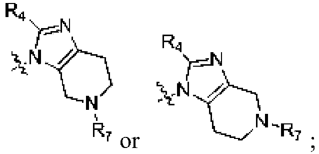
R₇ is selected from hydrogen, C(=O)R₈, C(=O)OR₈, C(=O)NR₈R₉ and SO₂R₈; preferably, R₇ is selected from C(=O)R₈, C (= O)OR₈ and SO₂R₈;
R₈ and R₉ are each independently selected from hydrogen and the following groups which are unsubstituted or substituted with 1-3 substituents: C1~C4 linear or branched alkyl, C1~C4 linear or branched haloalkyl, C3~C7 cycloalkyl, and benzyl, wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, and amino, preferably selected from halogen, hydroxyl, methoxy, ethoxy, methyl, ethyl, trifluoromethyl, trifluoromethoxy, and amino; preferably, R₈ and R₉ are each independently selected from hydrogen, C1~C4 linear or branched alkyl, C1~C4 linear or branched haloalkyl, and C3~C7 cycloalkyl; preferably, R₈ and R₉ are each independently selected from methyl, ethyl, n-propyl, cyclopropyl, isopropyl, n-butyl, sec-butyl, and tert-butyl.

In another preferred embodiment, the active ingredient is a compound selected from the following group, or a pharmaceutically acceptable salt, a solvate, an optically pure isomer, a stereoisomer, or a mixture thereof:

| Number | Structure | Number | Structure |
|---|---|---|---|
| DC521020 | 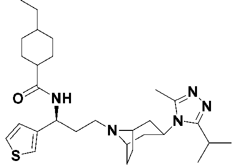 | DC521022 | 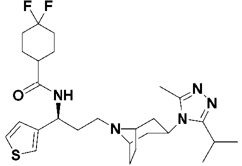 |
| DC521026 | 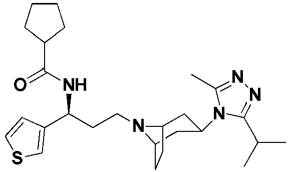 | DC521042 | 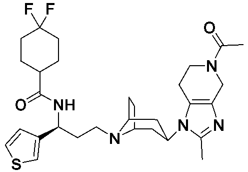 |
| DC521043 | 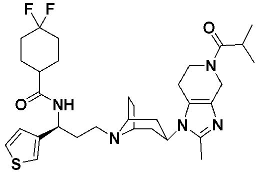 | DC521068 | 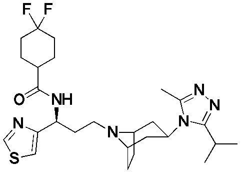 |
| DC521124 | 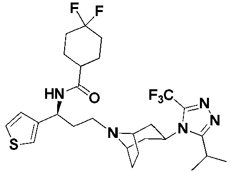 | DC521127 | 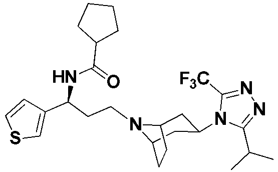 |
| DC521131 | 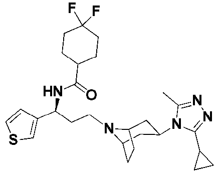 | DC521133 | 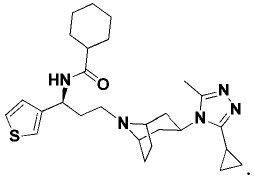 |

### Indications

Multiple sclerosis (MS) is an immune-mediated chronic, disabling inflammatory demyelinating disease of the central nervous system. It commonly involves the periventricular region, juxtacortical areas, optic nerve, spinal cord, brainstem, and cerebellum The disease is characterized by spatial dissemination and temporal dissemination of lesions. Spatial dissemination refers to lesions that occur in multiple locations, including the brain, brainstem, cerebellum, and spinal cord, which can be simultaneously or sequentially affected. Temporal dissemination refers to a remission-relapse course; due to the wide range of affected areas and repeated relapses, it can ultimately lead to disability or even death. The clinical manifestations are diverse, with common symptoms including decreased vision, diplopia, sensory disturbances in limbs, motor disturbances in limbs, ataxia, and bladder or rectal dysfunction.

### Relapsing-remitting multiple sclerosis (RRMS)

Relapsing-remitting multiple sclerosis is characterized by relapses, for example, they are defined as a new neurological deficit or neurological worsening episode lasting more than 24 hours, without fever or infection. There is no significant disease progression during remission. At different time points, RRMS can be further characterized as active (with evidence of recurrence and/or new MRI activity) or inactive, and as worsening (with confirmed increase in disability within a specific time period after recurrence) or non-worsening.

### Primary progressive multiple sclerosis (PPMS)

The term RMS (Relapsing Multiple Sclerosis) encompasses RRMS, secondary progressive multiple sclerosis (SPMS), and clinically isolated syndrome (CIS).

PPMS is characterized by worsening neurological function (disability accumulation) from the onset of symptoms, without early relapse or remission. PPMS can be further characterized at different time points as active (with evidence of occasional recurrence and/or new MRI activity) or inactive, and progressive (with evidence of disease worsening measured objectively over time, with or without recurrence or new MRI activity) or non-progressive. PPMS can have a brief period of disease stability with or without recurrence or new MRI activity, and the periods when disability increase occurs with or without new recurrence or MRI lesions.

### Secondary progressive multiple sclerosis (SPMS)

Secondary progressive multiple sclerosis develops after an initial relapse-remission course. Most people diagnosed with RRMS will eventually progress to a secondary progressive course, in which progressive deterioration of neurological function (disability accumulation) occurs over time. SPMS can be further characterized at different time points as active (with evidence of recurrence and/or new MRI activity) or inactive, and progressive (with evidence of disease worsening measured objectively over time, with or without recurrence) or non-progressive. During SPMS, occasional recurrence and stable periods may occur.

### Clinically isolated syndrome (CIS):

Clinically isolated syndrome (CIS) can refer to suggest a single clinical episode of central nervous system (CNS) inflammatory demyelination symptoms of multiple sclerosis (MS). CIS manifestations can be unifocal or multifocal and may typically involve the optic nerve, brainstem, cerebellum, spinal cord, or cerebral hemispheres.

The main advantages of the present invention include:
(a) The compound of the present invention has excellent repairing effects on spinal cord demyelination in multiple sclerosis.
(b) The compound of the present invention can significantly reduce the infiltration of immune cells and myeloid skewing in the spinal cord of multiple sclerosis.
(c) The compound of the present invention has a rapid therapeutic effect of multiple sclerosis and a significant effect in relieving the symptoms of the disease.
(d) The compound of the present invention can delay the progression of multiple sclerosis.
(e) The compound of the present invention has pharmacological properties and is suitable for long-term administration.

It should be understood that these examples are for illustrative purposes only and are not intended to limit the scope of the invention. Experimental methods in the following examples, unless otherwise specified, are generally performed under conventional conditions as described in Sambrook et al., Molecular Cloning: A Laboratory Manual (New York: Cold Spring Harbor Laboratory Press, 1989), or as recommended by the manufacturer. Percentages and parts are calculated by weight unless otherwise stated.

Unless otherwise defined, all technical and scientific terms used herein have the same meaning as are familiar to those skilled in the art. Furthermore, any methods and materials similar to or equivalent to those described herein may be applied to the methods of the present invention. The preferred implementation methods and materials described herein are for illustrative purposes only.

### Example 1. Preparation of compound DC521022

The synthesis route of Compound DC521022 is as follows:

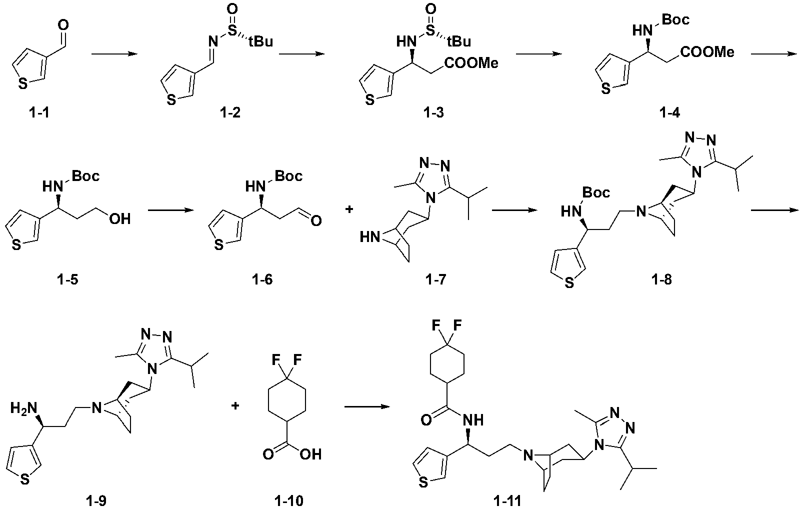

Compound 1-9 was synthesized using the above route. Compound 1-9 (35.6 mg, 0.1 mmol) were dissolved in 5 mL of dichloromethane, and then triethylamine (28 µL, 0.2 mmol), 4,4-difluorocyclohexanecarboxylic acid (18 mg, 0.11 mmol), HOBt (14.8 mg, 0.11 mmol), and EDCI (21.1 mg, 0.11 mmol) were added sequentially. The mixture was stirred at room temperature and reacted for 12 hours. Water was added, and the mixture was extracted with dichloromethane, washed with saturated brine, dried over anhydrous sodium sulfate, concentrated, and separated by column chromatography (DCM: CH₃OH = 10:1) to obtain the final product Compound DC521022.

¹HNMR (500 MHz, CDCl₃): *δ* 7.31 (dd, *J =* 5.0, 2.9 Hz, 1H), 7.13 (d, *J =* 2.9 Hz, 1H), 7.01 (dd, *J* = 4.9, 1.4 Hz, 1H), 4.29 (tt, *J* = 12.0, 5.5 Hz, 1H), 3.41 (s, 2H), 2.98 (p, *J =* 6.8 Hz, 1H), 2.51 (s, 5H), 2.37-1.97 (m, 10H), 1.97-1.55 (m, 11H), 1.38 (dd, *J* = 6.8, 2.1 Hz, 6H). ¹³C NMR (125 MHz, CDCl₃) *δ* 173.25, 159.11, 150.61, 142.95, 126.53, 126.12, 121.04, 77.23, 59.02, 58.40, 47.99, 47.63, 47.18, 42.93, 35.52, 35.34, 34.51, 33.02, 32.82, 26.72, 26.03, 25.96, 25.86, 21.67, 13.21. ESI-MS m/z: 520.3 [M + H] ⁺. HRMS (ESI) calcd for C₂₇H₃₉F₂N₅OS ([M + H]⁺): 520.2916; found: 520.2917.

### Example 2. Test of the onset of multiple sclerosis in vivo

Autoimmune encephalomyelitis (EAE) was an animal model of multiple sclerosis. In this experiment, behavioral scores and weight assessments were performed on two groups of mice daily until the end of the experiment to investigate the effect of compound DC521022 on the onset of multiple sclerosis.

Experimental animals: C57/BL6 mice, 10 weeks old, female, randomly divided into experimental group and control group: model control group (EAE + Vehicle): administered saline after modeling; model administration group (EAE + DC521022): administered DC521022 suspension after modeling. According to the different administration times and methods, both the model control group and model administration group were further divided into four groups: intraperitoneal injection of 50 mg/kg DC521022 after immunization; gavage administration of 50 mg/kg DC521022 after immunization; intraperitoneal injection of 50 mg/kg DC521022 at the onset of symptoms; and gavage administration of 50 mg/kg DC521022 at the onset of symptoms. The control group was given the same dose of saline at the same time.

Preparation method of DC521022: According to the preparation plan, an appropriate amount of DC521022 was weighed for use. First, an appropriate amount of solvent was added to a beaker marked with a certain volume. Then an appropriate amount of hydrochloric acid (the formula for calculating the amount of hydrochloric acid: hydrochloric acid: test sample = 1:1 (molar ratio)) was added to the beaker. After the test sample was added slowly, the mixture was shaken rapidly and continuously for about 16 hours at room temperature until the test sample was completely dissolved and the solution was clear. The solvent was added to the corresponding scale line.

Animal experiment method: mouse polypeptide antigen MOG35-55 (purity>95%) was repeatedly mixed with complete Freund's adjuvant containing inactivated Mycobacterium tuberculosis in a syringe for emulsification to form a water in oil state. The injection site was selected on the back skin at the midpoint between the bilateral upper and lower limbs of mice. 100 µL of the emulsion (200 µg of MOG35-55 (CAS No.: 149635-73-4), 500 µg of inactivated Mycobacterium tuberculosis) was injected subcutaneously on each side, for a total of 200 µL. On the day of immunization and two days later, 200 ng of pertussis toxin was injected intraperitoneally. After the animal experiment was completed, the mice's condition was observed daily, and clinical scoring was performed and recorded. Scores were based on 0-5 points: 0: asymptomatic; 1: tail weakness; 2: hind limb weakness or unstable gait; 3: complete hind limb paralysis; 4: complete hind limb paralysis with forelimb weakness or paralysis; 5: death.

Explanation of successful modeling: after the above modeling, the mice showed tail drooping symptoms, indicating that the mice were successfully induced with autoimmune encephalomyelitis (EAE) by MOG35-55 immunization.

Administration method: model administration group (EAE+DC521022): C57/BL6 mice were immunized with MOG35-55 to induce EAE, and DC521022 was administered via intraperitoneal injection and gavage on the day of modeling and the first day of disease onset, respectively; model control group (EAE+Vehicle): C57/BL6 mice were immunized with MOG35-55 to induce EAE, and the same volume of saline was administered via intraperitoneal injection and gavage on the day of modeling and the first day of disease onset, respectively.

Experimental method: after modeling, DC521022 suspension was immediately administered via intraperitoneal injection and gavage on the day of immunization and the first day of disease onset, respectively. The control group mice were given saline. Administration was repeated daily, and the mice's condition was observed and clinically scored until Day 14 and Day 28 after modeling. At the peak of EAE and two weeks after treatment, myeloid skewing in the bone marrow, spinal cord demyelination, and inflammatory cell infiltration in the spinal cord were analyzed.

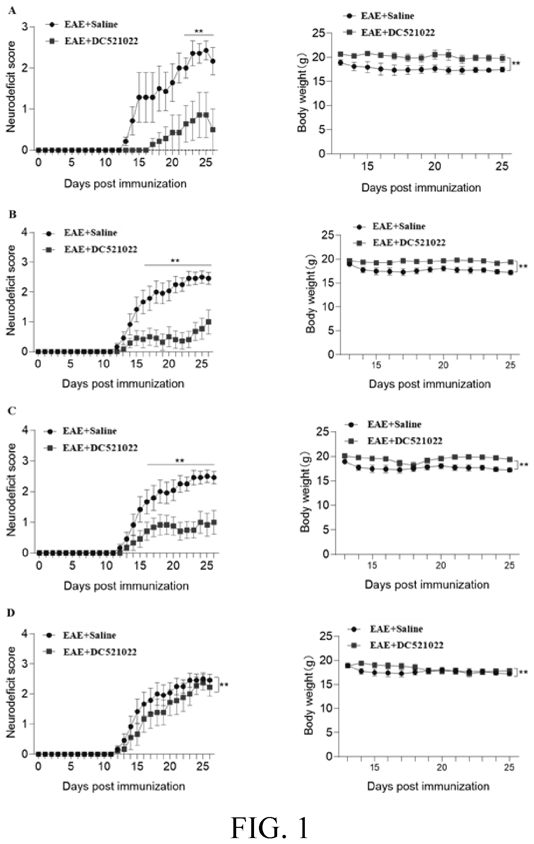

Experimental results showed after treatment with DC521022, the onset of symptoms in mice was later than the control group and the clinical scores were lower. DC521022 treatment also improved the weight of mice, compared to the control group, the weight of mice after treatment increased (as shown in Figure 1). This type of compound has promising prospects for the treatment of multiple sclerosis and therefore has great commercial value.

### Example 3. Test of spinal cord demyelination levels in multiple sclerosis in vivo

In this experiment, immunohistochemistry was used to perform fast blue staining of mouse spinal cord to compare the level of spinal cord demyelination in the DC521022 treatment group and the saline treatment group 14 days and 28 days after successful EAE modeling.

Preparation of paraffin section: on the first day after successful induction of the EAE model, the mice were divided into experimental group and control group. Two groups in the experimental group were injected with DC521022 intraperitoneally or by gavage on the day of modeling, and the other two groups were injected with DC521022 intraperitoneally or by gavage at the onset of EAE symptoms. The control group was injected with the same dose of saline in the same way. The mice administrated on the day of immunization were observed for 2 weeks, and the mice administrated at the onset of symptoms were treated for 2 weeks. After anesthesia, the sample was collected and the spinal cord was fixed after being perfused with phosphate buffer and 4% paraformaldehyde in sequence. The mice were first deeply anesthetized with 20 µg/g tribromoethanol and properly fixed. The skin was longitudinally cut along the line connecting the chest and abdomen to expose the thoracic and abdominal cavities. The heart was then freed and the liver was cut to open the venous channel. 20 mL of PBS and 20 mL of 4% paraformaldehyde were perfused sequentially through the left ventricle. The standard for successful perfusion was that the liver and other abdominal organs turned white. After perfusion, the skin was cut along the midline of the back, the spine and the spinal cord was dissected, from the thoracic vertebrae to the coccyx. Then, the spine was cut along one side to expose the spinal cord. The lumbar spinal cord segment was taken, and fixed and immersed in 4% paraformaldehyde at 4 °C for more than 16 h. After fixation, the brain tissue was removed from formaldehyde, placed in a tissue embedding cassette and labeled. It was then sequentially immersed in 50% ethanol, 75% ethanol, 85% ethanol, 95% ethanol, and anhydrous ethanol (twice) for 1 hour each, followed by xylene for 20 minutes (twice), and soft paraffin (52-54 °C) and hard paraffin (56-58 °C) for 1.5 hours each. The specimens were then embedded after undergoing dehydration, clearing, and paraffin infiltration in the above solutions sequentially. After embedded, the experimental group and control group specimens were taken out, and the spinal cord tissue was cut into 5 µm thick sections using a paraffin microtome. The sections were fully unfolded in distilled water at 40 °C, mounted onto slides, dried on a slide warmer at 60 °C for 2 hours. The paraffin sections were stored at room temperature.

Immunohistochemical staining steps: spinal cord sections were sequentially placed in xylene 5 (twice), anhydrous ethanol for 5 min (twice), 95% ethanol, 85% ethanol, 75% ethanol, 50% ethanol, and ultralight water (DDW) for 2 min each. They were then put in a staining box containing Luxol Fast Blue Solution and placed in a 60°C incubator for 2 h. After thorough rinsed with distilled water for 5 min, they were repeatedly immersed in lithium carbonate with each time for no more than 20 s. This process was repeated with Alcohol Reagent, and they were rinsed while observed under a microscope until the gray matter was colorless and the white matter remained blue. They were then rinsed again with distilled water, placed in a Cresyl Echt Violet kit for 2~5 minutes, quickly rinsed in distilled water, and then rapidly dehydrated in anhydrous ethanol. Finally, they were mounted with synthetic resin and photographed as soon as possible under a regular microscope. The staining results of the images were analyzed using imageJ software. The demyelinated area and white matter area of each spinal cord segment in six sections were averaged to calculate the percentage of demyelinated area relative to the total area.

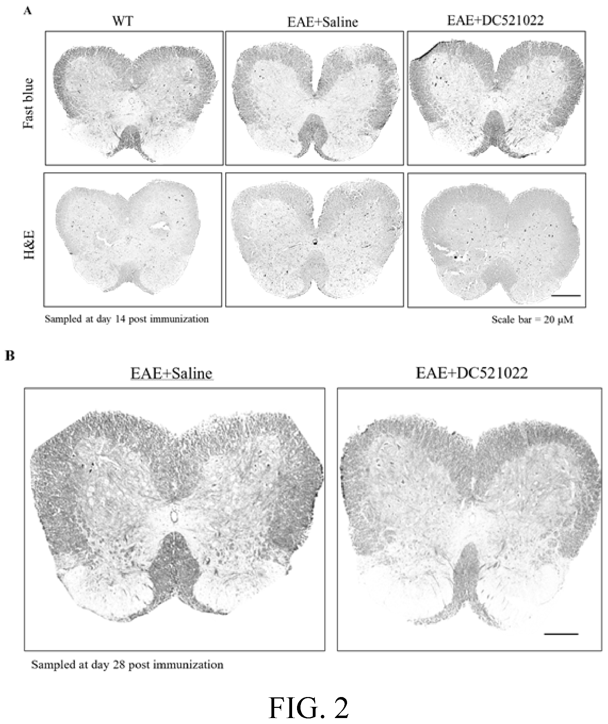

The results showed that, compared with the control group, the demyelination of mice treated with DC521022 improved after 14 and 28 days of administration, and it had a significant repair effect on spinal cord demyelination (Figure 2).

### Example 4. Test of the level of infiltrating immune cells in the spinal cord of multiple sclerosis in vivo

In this experiment, immunohistochemistry was used to perform HE staining on the spinal cord of mice to compare the levels of infiltrating immune cells in the spinal cord of the DC521022 treatment group and the saline treatment group 14 and 28 days after the successful EAE modeling.

The steps for preparing paraffin sections are the same as those for Fast Blue staining.

Immunohistochemical staining steps: spinal cord sections were sequentially placed in xylene for 5 min (twice), anhydrous ethanol for 5 min (twice), 95% ethanol, 85% ethanol, 75% ethanol, 50% ethanol, and DDW for 2 min each. They were stained with hematoxylin staining solution for 5 min, rinsed with tap water for 5 s; differentiated with differentiation solution for 3 s, rinsed with tap water for 20 s to remove the differentiation solution; blued with bluing solution for 30 s, rinsed with tap water for 20 s to remove the bluing solution; stained with eosin for 30 s, rinsed with tap water for 5 s, dehydrated and cleared, and the sections were mounted.

The staining results of the images were analyzed using ImageJ software. The inflammatory cell infiltration and white matter area of each spinal cord segment in six sections were averaged to calculate the percentage of inflammatory cell infiltration area relative to the total area.

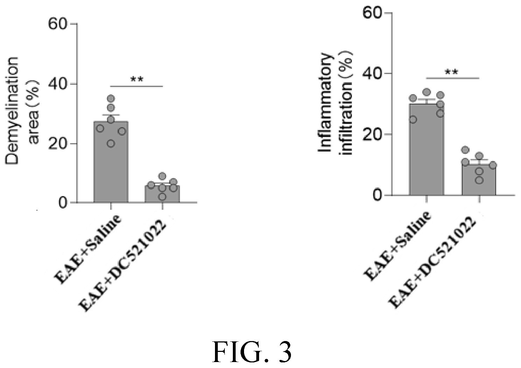

The experimental results showed that after long-term treatment with DC521022, the infiltrating immune cells in the spinal cord of mice decreased, with statistically significant differences (Figure 3).

### Example 5. Test of myeloid skewing in multiple sclerosis in vivo

In this experiment, flow cytometry was used to investigate the myeloid skewing in the bone marrow of mice in each group of the model on days 14 and 28 of the experiment after treatment with compound DC521022.

Preparation of brain tissue single-cell suspension: mice were deeply anesthetized by intraperitoneal injection of tribromoethanol and properly fixed. The left femur of the mice was taken and soaked in ice-cold PBS for later use. The bone marrow of femur of mice was blown into a 15 mL centrifuge tube, centrifuged at 350 G for 5 min, and the supernatant was discarded. 1 mL of erythrocyte lysis buffer was added, and lysis was carried out for 6 min. Then lysis was terminated by adding 5 mL of PBS, and it was centrifuged at 350 G for 5 min, and then resuspended in 1 mL of PBS. Cells were counted.

The following channels were selected for the color matching of fluorescent dyes used in flow cytometry antibody staining: fluorescein isothiocyanate (FITC), Phycoerythrin (PE), PerCP-Cy5.5, allophycocyanin (APC), allophycocyanin-anthocyanin 7 (APC-Cy7), and phycoerythrin-anthocyanin 7 (PE-Cy7).

Specific flow cytometry antibodies against mouse target antigens: anti-mouse lin-antibody, anti-mouse sca-1 antibody, anti-mouse ckit antibody, anti-mouse CD48 antibody, anti-mouse CD150 antibody, anti-mouse CD127 antibody, anti-mouse CD135 antibody, anti-mouse CD34 antibody, anti-mouse 16/32 antibody, anti-mouse CD115 antibody, and anti-mouse 7CD135 antibody.

Surface antibody staining: surface antibodies were added to a flow cytometry tube containing 100 µL of single-cell suspension and vortexed to mix (added sequentially strictly according to the manufacturer's recommended dosages). After addition, the mixture was incubated at 4°C in the dark for 30 min. 1 mL of PBS was added for washing, followed by centrifugated at 1500 rpm for 5 min. The supernatant was discarded, and the precipitate was resuspended in 400 µL PBS and mixed well. The sample was then filtered and loaded onto the flow cytometer.

In this experiment, flow cytometry antibody staining was performed using blank control, single positive compensation, isotype control, and fluorescence minus one (FMO) control. FACS Aria II was used to collect flow cytometry data, and FlowJoV10 (Version 10, FlowJo, LLC) was selected for analysis of the flow cytometry data.

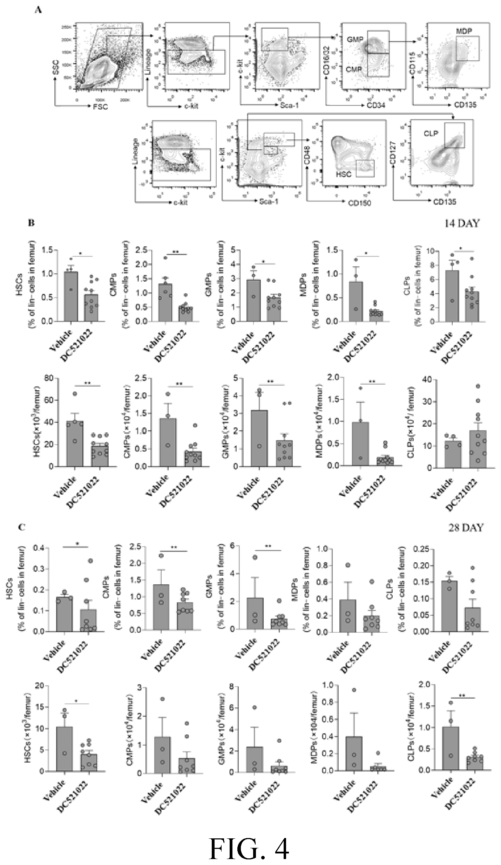

The experimental results showed that the proportion and quantity of bone marrow hematopoietic stem cells in EAE mice treated with DC521022 were significantly reduced on Day 14 and Day 28, and the myeloid skewing was significantly decreased (Figure 4).

In summary, long-term administration of the test substance DC521022 can delay the onset of multiple sclerosis in EAE model mice, has a significant repair effect on demyelination in mice, and reduces infiltrating immune cells and myeloid skewing in the spinal cord.

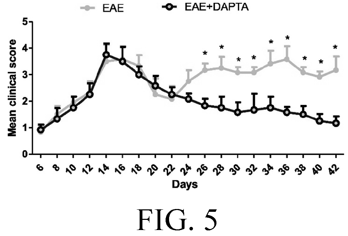

Compared to the effects of the reported selective CCR5 antagonist DAPTA (see Figure 5 or Sheikh et al., Cellular Immunology. 2022, 379, 104580, Figure 1), administration of the drug after onset of the disease on day 6 did not delay the onset of the disease and even aggravated the progression of the disease. The therapeutic effect gradually became apparent after 22 days of administration.

The therapeutic effect of the present invention on EAE mice is significantly superior to that studied in Sheikh et al. The present invention uses two drug administration models for research. Drug administration on the day of modeling significantly delayed the onset of disease in EAE mice, with lower behavioral scores and better body weight compared with the control group (Figures 1A and 1C). Drug administration after symptoms appeared in mice significantly delayed disease progression. Simultaneously, the behavioral scores were lower and body weight improved significantly (Figures 1B and 1D).

All literatures mentioned in the present application are incorporated herein by reference, as each one is individually incorporated by reference. In addition, it should also be understood that, after reading the above teachings of the present invention, those skilled in the art can make various changes or modifications, and these equivalents also fall in the scope defined in the appended claims.

## Claims

1. Use of a compound of formula (I), or a pharmaceutically acceptable salt, a solvate, an optically pure isomer, a stereoisomer, or a mixture thereof, wherein the compound is used for the preparation of a medicament for treating and/or preventing multiple sclerosis and a related disease thereof;
the compound of formula (I) has the following structure: 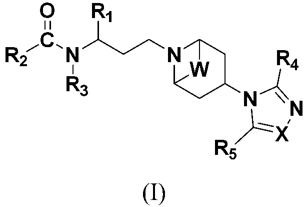
wherein,
W is absent or is -CH₂CH₂-; X is N or CR₆;
R₁ is selected from a 5~7 membered heteroaryl which is unsubstituted or substituted with 1-3 substituents, wherein the heteroaryl contains 1~3 heteroatoms selected from oxygen, sulfur, and nitrogen, and the substituent is each independently selected from halogen, C1~C4 linear or branched alkyl, C1~C4 linear or branched haloalkyl, C1~C4 linear or branched alkoxy, C1~C4 linear or branched haloalkoxy, -NR₁₀R₁₁, -C(=O)R₁₂, C1~C4 linear or branched alkanoyloxy, cyano, nitro, and hydroxyl, or two adjacent substituents, together with the carbon atoms to which they are attached, form a 5-7 membered ring;
R₁₀ and R₁₁ are each independently selected from H, C1~C4 linear or branched alkyl and - C(=O)R₁₃;
R₁₂ is selected from C1~C4 linear or branched alkyl, C1~C4 linear or branched alkoxy, hydroxy, amino (NH₂) and C1~C4 linear or branched alkylamino;
R₁₃ is selected from H and C1~C4 linear or branched alkyl;
R₂ is selected from the following groups which are unsubstituted or substituted with 1-3 substituents: C1~C6 linear or branched alkyl, C3~C7 cycloalkyl, 4~7 membered heterocyclyl, C6~C12 aryl, or 5~7 membered heteroaryl; wherein the substituent is selected from halogen, hydroxyl, C1~C4 linear or branched alkyl, C1~C4 linear or branched haloalkyl, C1~C4 linear or branched alkoxy, C1~C4 linear or branched alkyl carbonyl, C1~C4 linear or branched haloalkoxy, C1~C4 linear or branched alkylsulfonyl, C1~C4 linear or branched alkylsulfonyl carbamoyl, tetrazolyl, cyano, nitro, amino, carboxyl, phenyl and phenoxy;
R₃, R₄ and R₅ are each independently selected from hydrogen, C1~C6 linear or branched alkyl, C1~C6 linear or branched haloalkyl and C3~C7 cycloalkyl;
R₆ is selected from hydrogen and C1~C6 linear or branched alkyl;
or, R₅ and R₆ can be connected together with 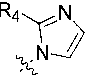
to form 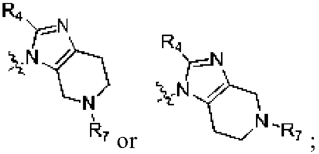
R₇ is selected from hydrogen, C(=O)R₈, C(=O)OR₈, C(=O)NR₈R₉, SO₂R₈ and the following groups substituted with 1-3 substituents: C1~C6 linear or branched alkyl, C3~C7 cycloalkyl, 4~7 membered heterocyclyl, benzyl, C6~C12 aryl and 5~7 membered heteroaryl; wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino, carboxyl;
R₈ and R₉ are each independently selected from hydrogen, and the following groups which are unsubstituted or substituted with 1-3 substituents: C1~C6 linear or branched alkyl, C3~C7 cycloalkyl, 4~7 membered heterocyclyl, benzyl, C6~C12 aryl and 5~7 membered heteroaryl; wherein the substituent is selected from halogen, hydroxyl, C1~C4 linear or branched alkoxy, C1~C4 linear or branched alkyl, C1~C4 linear or branched haloalkyl, C1~C4 linear or branched haloalkoxy, cyano, nitro, amino, carboxyl.

2. The use according to claim 1, wherein the multiple sclerosis is selected from the group consisting of: relapsing-remitting multiple sclerosis, secondary progressive multiple sclerosis, primary progressive multiple sclerosis, progressive-relapsing multiple sclerosis, or a combination thereof.

3. The use according to claim 1, wherein the multiple sclerosis related disease is selected from the group consisting of: neuromyelitis optica, acute disseminated encephalomyelitis, stroke, craniocerebral injury, epilepsy, Alzheimer's disease, Parkinson's disease, immune inflammatory response, and demyelinating injury.

4. The use according to claim 1, wherein the multiple sclerosis and a related disease thereof comprises: relapsing-remitting multiple sclerosis, secondary progressive multiple sclerosis, primary progressive multiple sclerosis, stroke, craniocerebral injury, epilepsy, immune inflammatory response, and demyelinating injury.

5. The use according to claim 1, wherein the compound is used for:
(1) repairing demyelination in the spinal cord of multiple sclerosis;
(2) reducing infiltrating immune cells in the spinal cord of multiple sclerosis; and
(3) reducing myeloid skewing in the spinal cord of multiple sclerosis.

6. The use according to claim 1, wherein the compound of formula (I) is selected from the group consisting of:
| Number | Structure | Number | Structure |
|---|---|---|---|
| DC521020 | 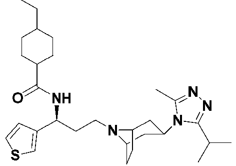 | DC521022 | 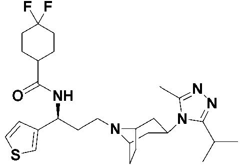 |
| DC521026 | 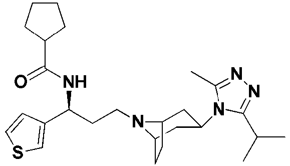 | DC521042 | 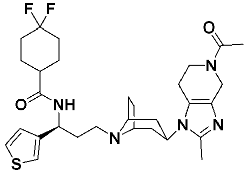 |
| DC521043 | 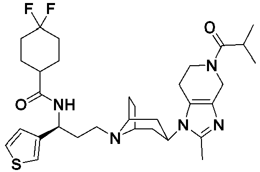 | DC521068 | 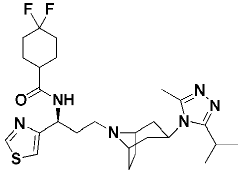 |
| DC521124 | 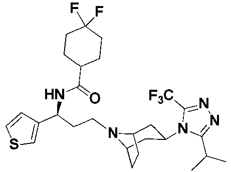 | DC521127 | 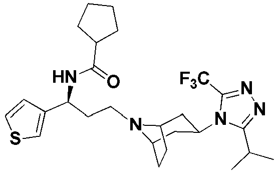 |
| DC521131 | 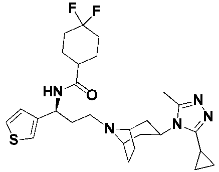 | DC521133 | 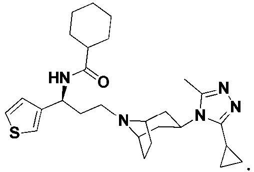 |

7. The use according to claims 1-6, wherein the administration method of the compound of formula (I) is selected from the group consisting of oral, rectal, and parenteral administration.

8. The use according to claim 7, wherein the method of the parenteral administration is selected from the group consisting of intravenous, intramuscular, or subcutaneous administration.

9. The use according to claim 7, wherein the formulation of the oral administration is selected from the group consisting of capsules, tablets, pills, powders, granules, emulsions, solutions, suspensions, syrups, and tinctures.

10. Use of a pharmaceutical composition, wherein the pharmaceutical composition is used for the preparation of a medicament for treating and/or preventing multiple sclerosis and a related disease thereof;
and the pharmaceutical composition comprises:
(i) a first active ingredient, wherein the first active ingredient is selected from the group consisting of: the compound of formula (I), or a pharmaceutically acceptable salt, a solvate, an optically pure isomer, and a stereoisomer thereof;
(ii) optionally, a second active ingredient, wherein the second active ingredient is selected from the group consisting of: interferon β-1a, interferon β-1b, teriflunomide, fingolimod, copaxone, ofatumumab, or a combination thereof; and
(iii) a pharmaceutically acceptable carrier;
the compound of formula (I) is as defined in claim 1.
